# DEMANDE DE BREVET EUROPEEN

(11) **EP 4 147 725 A1**
(43) Date de publication de la demande: **15.03.2023**
(21) Numéro de dépôt: 22192079.6
(22) Date de dépôt: 25.08.2022
(51) Int. Cl.: A61L 2/06, A61L 2/07, B27K 5/00, B65D 88/00

(54) **PROCÉDÉ POUR LE TRAITEMENT PHYTOSANITAIRE DU BOIS PAR LA VAPEUR, NOTAMMENT DANS UN CONTENEUR DE TRANSPORT MARITIME, ET SYSTÈME ASSOCIÉ**

(30) Priorité: 13.09.2021 FR 2109584
(71) Demandeur: Rentokil Initial, 93240 Stains (FR)
(72) Inventeur: VACQUER, Benoît, 76130 MONT SAINT AIGNAN (FR)
(74) Mandataire: Mazabraud, Xavier

(57) **Abrégé**

L'invention porte sur un procédé de traitement à la vapeur de grumes 8 dans un conteneur 1 qui comprend un sol 2 et deux parois latérales 4, le procédé comprenant la fourniture d'au moins deux buses d'angle 14, leur disposition dans un angle formé par une paroi latérale 4 respective avec le sol, et, leur orientation vers le fond du conteneur 2. L'invention porte aussi sur un système 9 de traitement, notamment un système comprenant un portique 10 qui comprend deux tuyères 14 formant les buses d'angle et des rampes 11,12 portant d'autres buses 16.

## Description

La présente invention se rapporte au domaine des systèmes et procédés de traitement phytosanitaire ou biocide par la chaleur. Ils s'appliquent au traitement des produits végétaux, notamment de marchandises contenant du bois, notamment des billes de bois ou grumes, mais pas uniquement. L'invention concerne plus particulièrement le traitement de quarantaine des conteneurs de transport maritime, notamment les conteneurs à la norme internationale ISO en vue d'effectuer des désinfections et des désinsectisations, des traitements d'ordres généraux d'éliminations parasitaire biocides.

Depuis le 1er mars 2005, en application de la Directive 2004/102/CE et de l'Arrêté du 9 novembre 2004, la norme NIMP15 de la FAO, relative aux matériaux d'emballage en bois utilisés dans le commerce international, ce type de traitement est obligatoire pour les emballages entrant dans l'Union Européenne en provenance de pays extérieurs à l'Union. Cette norme a pour but de prévenir les risques de diffusion en Europe d'organismes nuisibles véhiculés par le bois d'emballage, tout en limitant les entraves au commerce international. La norme prévoit deux types de traitements du bois :
- La fumigation au bromure de méthyle,
- Le traitement par la chaleur, consistant à soumettre le bois à une température de 56° C à cœur pendant au moins 30 minutes.

L'usage du bromure de méthyle ayant été interdit au sein de l'Union Européenne, seul subsiste le traitement par la chaleur.

Certains procédés de traitement par la chaleur utilisent des rampes disposées sous le toit du conteneur et qui distribuent de la vapeur vers le vas, sur les marchandises. De telles rampes peuvent aisément être disposées après le chargement des marchandises dans le conteneur, puis retirées, sans modifier le chargement. Cependant, ces procédés ne permettent pas un traitement uniforme du chargement.

Un but de l'invention, est de proposer un procédé et un système associé qui permettent un traitement biocide homogène d'une marchandise dans un conteneur de transport.

Pour résoudre ce problème l'invention propose un procédé pour un traitement thermique à l'aide d'un gaz d'une marchandise dans un conteneur qui comprend un sol, un toit, deux parois latérales et longitudinales, opposées entre elles, un fond, disposé transversalement entre les parois latérales, et, une entrée opposée audit fond, cette entrée comprenant de préférence une porte droite et une porte gauche, ce procédé comprenant la fourniture d'au moins deux buses d'angle et la disposition de chacune des buses d'angle à proximité d'un angle formé par une paroi latérale respective avec le sol.

Un tel procédé peut aussi comprendre la fourniture :
- d'au moins une buse basse et la disposition de cette buse basse à proximité du sol, entre les buses d'angle ; et/ou,
- d'au moins une buse haute et la disposition de cette buse haute à proximité du toit.

Avantageusement, les buses sont disposées de sorte qu'elles émettent un flux sensiblement horizontalement et longitudinalement en direction du fond du conteneur. De préférence, les buses sont disposées à proximité ou au voisinage immédiat d'un encadrement de l'entrée.

Les buses peuvent être disposées sur un portique qui assure une connexion fluidique entre elles et au moins un tuyau d'alimentation en gaz, de préférence de la vapeur d'eau, le portique étant disposé à l'intérieur du conteneur, à proximité de l'entrée.

Un procédé selon l'invention peut être appliqué au traitement thermique d'une marchandise qui comprend des grumes.

L'invention propose aussi un système pour la mise en œuvre d'un procédé selon l'invention.

Selon un premier mode de réalisation, un tel système peut comprendre un portique, ce portique comprenant au moins une rampe basse et une rampe haute fluidiquement reliées entre elles par un montant, chaque rampe comprenant au moins une buse, une buse d'angle étant fluidiquement reliée à la rampe base, de préférence à la jonction de cette rampe basse et du montant. Le portique peut aussi comprendre une rampe haute et deux rampes basses, chaque rampe basse étant fluidiquement reliée à une extrémité respective de la rampe haute par un montant respectif, une buse d'angle respective étant fluidiquement reliée à chaque rampe base, de préférence à la jonction de cette rampe basse et de son montant respectif. De préférence, chaque rampe comprend au moins trois buses.

Chaque buse d'angle est avantageusement formée à une extrémité distale d'une tuyère prévue pour s'étendre longitudinalement dans l'angle formé par le sol et une paroi latérale respective.

Le système peut en outre comprendre un panneau pour la fermeture du conteneur pendant le traitement, en remplacement d'une porte du conteneur maintenue ouverte. Le panneau comprend avantageusement au moins un passage pour au moins un tuyau pour alimenter le portique en gaz. Il peut aussi comprendre une bouche d'extraction.

Selon un deuxième mode de réalisation, un système de traitement pour la mise en œuvre d'un procédé selon l'invention comprend au moins une buse prévue pour être insérée dans un interstice autour d'une porte du conteneur. Cette buse comprend avantageusement un diffuseur prévu pour être inséré dans l'interstice et un emmanchement prévu pour y fixer un tuyau d'alimentation en gaz, de préférence de la vapeur, cet emmanchement étant prévu pour être à l'extérieur du conteneur. De préférence, la buse comprend en outre une chambre d'admission qui relie fluidiquement l'emmanchement et le diffuseur.

Plusieurs modes d'exécution de l'invention seront décrits ci-après, à titre d'exemples non limitatifs, en référence aux dessins annexés dans lesquels :
[Fig.1] est une vue schématique extérieure et de face d'un conteneur, portes ouvertes, équipé d'un premier mode de réalisation de l'invention comprenant un portique pour le traitement biocide de marchandises, ici des grumes, à l'aide de vapeur ;
[Fig.2] est une vue schématique intérieure de l'entrée du conteneur de la [Fig.1], porte de droite fermée, équipé du même portique de traitement biocide ;
[Fig.3] est une vue schématique extérieure de l'entrée du conteneur de la [Fig.1], porte de droite fermée, équipé d'un panneau amovible ;
[Fig.4] est une coupe schématique longitudinale du conteneur de la [Fig.1], illustrant une diffusion de la vapeur dans les marchandises ;
[Fig.5] est une coupe schématique longitudinale du conteneur de la [Fig.1], illustrant une diffusion de la vapeur dans les marchandises ;
[Fig.6] est une vue schématique extérieure et de face d'un conteneur, portes fermées, équipé d'un deuxième mode de réalisation de l'invention comprenant des buses pincées dans les joints des portes, pour le traitement biocide de marchandises à l'aide de vapeur ;
[Fig.7] est une vue schématique intérieure de l'entrée du conteneur de la [Fig.1], illustrant le mode de réalisation de la [Fig.6] ;
[Fig.8] est une vue schématique illustrant une buse droite pour le mode de réalisation des figures 6 et 7 ;
[Fig.9] est une vue schématique illustrant une buse inclinée pour le mode de réalisation des figures 6 et 7 ; et,
[Fig.10] est une vue schématique illustrant une buse démontable.

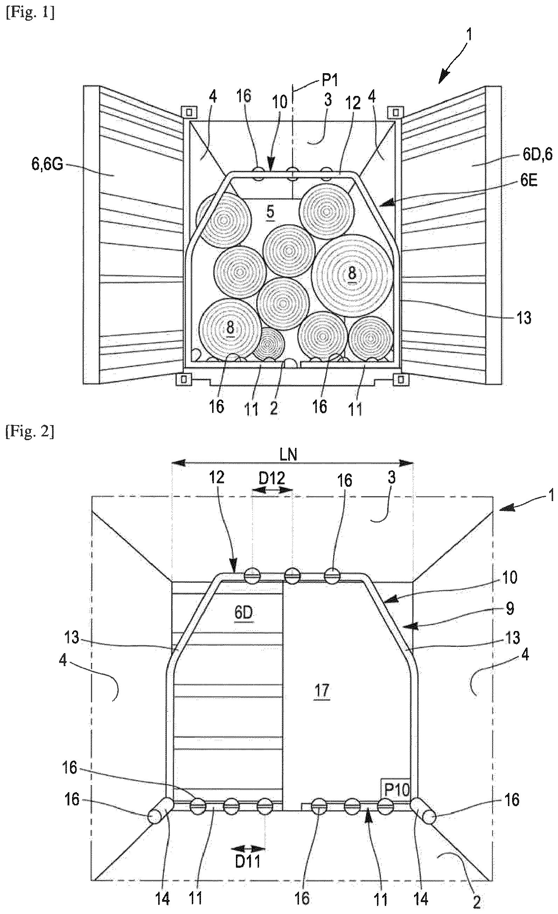

Les figures 1 à 5 illustrent un premier mode de réalisation pour l'invention.

La [Fig. 1] illustre un conteneur 1 de type multimodal de type « dry », vue de face et de l'extérieur. Le conteneur 1 est sensiblement symétrique par rapport à un plan vertical longitudinal P1. Il comprend un sol 2, un toit 3, deux parois latérales et longitudinales 4, opposées entre elles, un fond 5, disposé transversalement entre les parois latérales et une façade 6, opposée au fond 5. La façade comprend deux portes 6, une porte droite 6D, à droite sur la [Fig.1], et une porte gauche 6G, à gauche sur la même [Fig.1]. La façade constitue une entrée 6Edu conteneur, permettant son chargement. Dans l'exemple illustré, le conteneur contient une charge de grumes 8, qui y sont disposées longitudinalement.

Le conteneur 1 est équipé d'un système de traitement 9 comprenant un portique de traitement 10 selon l'invention. Le portique 10 est prévu pour diffuser un gaz. Dans l'exemple illustré, le gaz est de la vapeur d'eau.

Dans la position d'usage illustrée à la [Fig.2], le portique 10 est disposé dans l'encadrement de l'entrée 6E, de sorte que l'une au moins des portes 6 peut être fermée et verrouillée. Dans l'exemple décrit, comme illustré aux [Fig.2] et 3, c'est la porte droite 6D qui est fermée et verrouillée. Dans cette position d'usage, le portique est disposé contre la porte fermée 6D, à l'intérieur du conteneur 1.

Le portique 10 comprend deux rampes basses 11 et une rampe haute 12, toutes trois disposées horizontalement et parallèlement à la façade. Les rampes basses 11 sont disposées sur le sol 2 et y servent d'appuis pour le portique. Deux montants 13 relient fluidiquement chacun une rampe basse 11 respective avec la rampe haute 12. Les rampes basses sont fluidiquement reliées entre elles uniquement par un premier montant, la rampe haute et le deuxième montant.

A la jonction de chaque montant 13 avec la rampe basse 11 correspondante, le portique comprend une tuyère 14. Les tuyères sont disposées sensiblement perpendiculairement à un plan P10 défini par les buses, plan P10 qui, dans la position d'usage, est sensiblement parallèle à la porte fermée 6D. Dans la position d'usage, chaque tuyère s'étend longitudinalement le long d'une paroi latérale 4 respective, dans l'angle que cette paroi 4 forme avec le sol 2.

Chaque rampe 11, 12 comprend trois buses 16. Dans le mode de réalisation illustré, le portique comprend donc neuf buses et deux tuyères. Chaque tuyère forme, à son extrémité distale, une buse d'angle. Dans la position d'usage, buses et tuyères sont orientées de façon à générer des flux de vapeur sensiblement longitudinaux, c'est-à-dire dirigés vers le fond 5 du conteneur. Les tuyères mesurent plusieurs dizaines de centimètres de longueur ; les buses sont courtes relativement à la longueur des tuyères. Les buses des rampes basses et les tuyères sont écartées entre elles d'une distance D11 comprise entre le cinquième et le huitième d'une largeur intérieure LN du conteneur, de préférence environ le septième de la largeur LN. La largeur LN est mesurée entre les parois latérales 4.

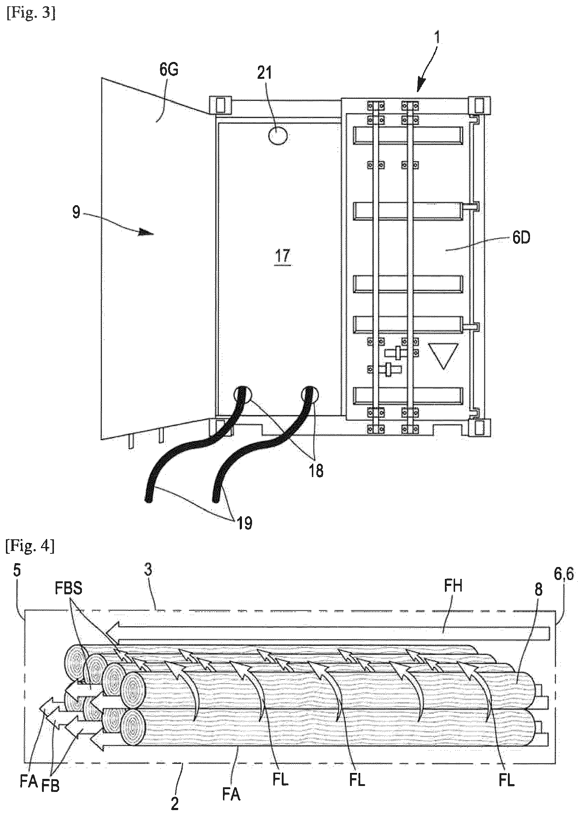

Comme particulièrement illustré à la [Fig.3], le système 9 comprend un panneau 17 pour la fermeture du conteneur 1 pendant le traitement, en remplacement de la porte gauche 6G qui est maintenue ouverte. Le panneau est percé de deux trous inférieurs, chacun pour le passage d'un tuyau souple 19. Chaque tuyau 19 est relié à une rampe basse 11 respective. Le panneau 17 comprend en outre un trou supérieur 21 formant une bouche d'extraction, prévue pour l'évacuation des gaz, notamment de la vapeur, en excès à l'intérieur du conteneur 1, pendant le traitement.

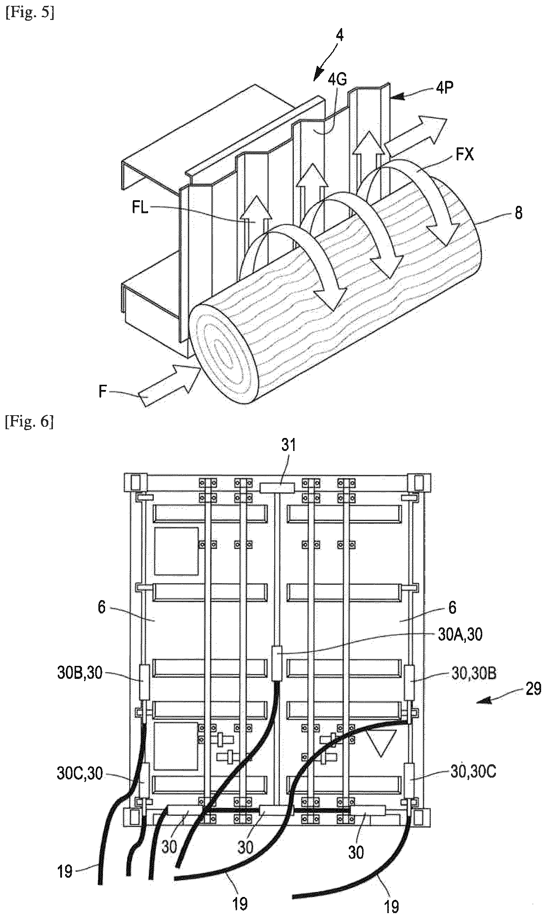

Les figures 4 et 5 illustrent la circulation des gaz dans le conteneur, au cours du traitement des grumes 8. Les buses 16 de la rampe haute 12 génèrent un flux haut FH qui se diffuse depuis l'entrée 6E vers le fond 5, entre le toit 3 et les grumes 8. Les buses 16 des rampes basses 11, génèrent un flux bas FB qui se diffuse le long du sol 2 dans les espaces entre les grumes 8 ; le flux bas engendre, par convection ou turbulences, des flux secondaires FBS qui parcourent de bas en haut les grumes 8. Chaque tuyère 14 génère un flux d'angle FA qui se diffuse le long d'un angle formé par le sol 2 et une paroi latérale 4 respective, dans l'espace laissé par les grumes 8 correspondant.

Comme particulièrement illustré à la [Fig.5], les parois latérales 4 du conteneur ont un parement 4P qui, en coupe horizontale, a sensiblement la forme d'une frise grecque et forme des gorges verticales 4G. Le flux d'angle FA engendre, par convection ou turbulence, des flux latéraux FL qui profitent des gorges 4G pour remonter le long des parois latérales, maintenant les parois latérales en température ; ils limitent le refroidissement du conteneur et y maintiennent une température plus homogène. Les flux latéraux FL se divisent, par convection ou turbulence, en flux auxiliaires FX qui enveloppent les grumes 8 les plus proches.

Grâce notamment à la présence des rampes basses 11 et des tuyères 14, le système de traitement permet une répartition de la vapeur et les températures à l'intérieur du conteneur plus homogène que les systèmes de l'art antérieur.

Les buses d'angle sont particulièrement avantageuses, puisqu'elles permettent d'améliorer l'homogénéité des températures dans le conteneur, donc d'améliorer le traitement des grumes 8.

On va maintenant décrire un deuxième mode de réalisation d'un système 29 de traitement thermique selon l'invention, en référence aux figures 6 à 10.

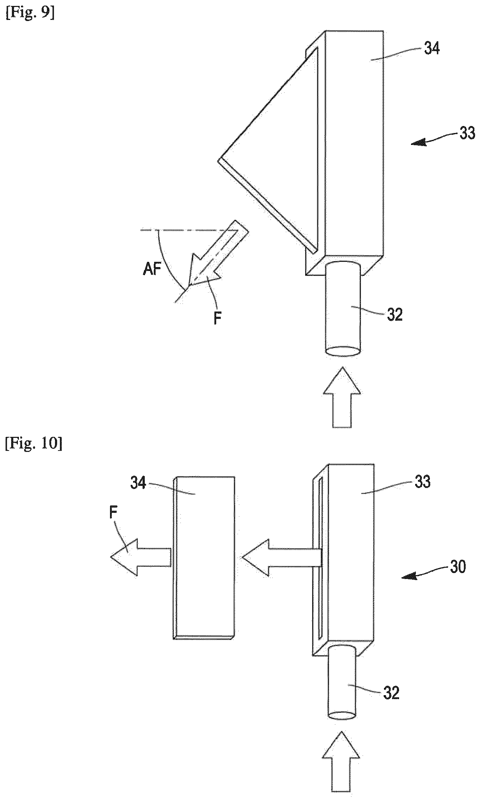

Dans ce mode de réalisation, le système 29 comprend un ensemble de huit buses 30. Les buses seront décrites plus loin en référence aux figures 9 à 10. Le système est utilisé avec les deux portes 6 fermées. Les buses 30 sont insérées dans les interstices autour des portes. Une buse 30A est insérée entre les deux portes, à mi-hauteur. Entre chaque porte 6 et la paroi latérale 4 correspondante, une buse latérale 30B est insérée à mi-hauteur et une buse d'angle 30C est insérée à proximité du sol. Entre les portes 6 et le sol 2, trois buses basses 30D sont insérées.

Les buses 30 sont alimentées en vapeur par des tuyaux souples 19. Les trois buses basses sont montées en série et alimentées par un même tuyau 19. Les autres buses sont indépendantes et chacune est alimentée en vapeur par un tuyau 19 respectif.

Le système 29 comprend en outre une bouche d'extraction 31, insérée entre les portes 6 et le toit 3. La bouche 31 est prévue pour l'évacuation des gaz, notamment de la vapeur, en excès à l'intérieur du conteneur 1 pendant le traitement. La bouche d'extraction 31 peut être une buse non reliée à un tuyau d'alimentation.

Comme dans le premier mode de réalisation, les flux de vapeur F sont dirigés vers le fond du conteneur 1. De la même façon, les buses d'angle sont particulièrement avantageuses, puisqu'elles permettent d'améliorer l'homogénéité des températures dans le conteneur, donc d'améliorer le traitement des grumes 8, de la même façon qu'illustré à la [Fig.5].

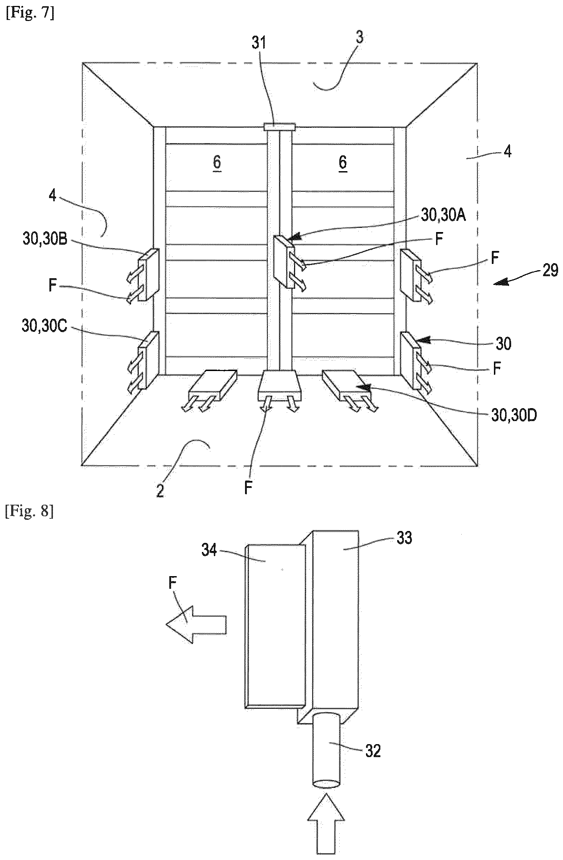

Les figures 8 à 10 illustrent des buses 30 selon l'invention. Chaque buse 30 comprend un emmanchement 32, pour y fixer un tuyau d'alimentation 19, une chambre d'admission 33 et un diffuseur 34. L'emmanchement 32 est prévu pour se fixer, par emboîtement, à un tuyau d'alimentation 1. Le diffuseur 34 est conçu pour être pincé dans un interstice. La chambre d'admission 33 sert de connexion fluidique entre l'emmanchement 32 et le diffuseur 34 ; elle est prévue pour être disposée à l'extérieur du conteneur, pendant le traitement.

Typiquement, un diffuseur a une ouverture, c'est-à-dire l'épaisseur de la lame d'air formée par le flux F à la sortie du diffuseur, comprise entre 2 et 4 millimètres.

Dans l'exemple illustré à la [Fig.8], le diffuseur est conformé pour que le flux F qui en sort soit orienté horizontalement et longitudinalement vers le fond 5 du conteneur.

Le diffuseur peut cependant être conformé afin que le flux ne soit pas dirigé horizontalement et longitudinalement. Dans l'exemple illustré à la [Fig.9], le flux est orienté vers le bas d'un angle AF d'environ quarante-cinq degrés.

De préférence, le diffuseur, la chambre et l'emmanchement sont d'une seule pièce.

Comme illustré à la [Fig.10], le diffuseur peut cependant être amovible. Ainsi, un tel diffuseur peut être abandonné, et éventuellement bouché, de sorte qu'il n'est pas utile d'ouvrir les portes 6 après le traitement. Ceci est particulièrement avantageux, notamment pour éviter que le traitement perde de son efficacité en cas d'ouverture. C'est aussi avantageux si des produits toxiques sont utilisés pour le traitement, nécessitant de maintenir le conteneur fermé, par exemple le temps que leur dangerosité soit atténuée.

Dans les deux modes de réalisation, une buse d'angle est disposée à une distance inférieure à quarante centimètres du sol 2 et de la paroi latérale 4, de préférence à une distance inférieure à vingt centimètres.

Bien sûr, l'invention n'est pas limitée aux exemples qui viennent d'être décrits. Au contraire, l'invention est définie par les revendications qui suivent.

Il apparaîtra en effet à l'homme de l'art que diverses modifications peuvent être apportées aux modes de réalisation décrits ci-dessus, à la lumière de l'enseignement qui vient de lui être divulgué.

Les deux modes de réalisation peuvent être combinés entre eux.

Ainsi, dans le deuxième mode de réalisation, d'autres buses, hautes, peuvent être insérées entre les portes et le toit. Aussi, une buse d'angle peut être introduite entre chaque porte et le plancher, à proximité de chaque paroi latérale, en complément ou à la place des buses d'angles précédemment décrites.

Le système de traitement selon l'invention permet de traiter des produits déjà chargés dans un conteneur, tels que des produits végétaux, grumes de bois ou des planches de bois. Il peut être installé a posteriori du chargement du bois dans le conteneur maritime, et non pas au préalable au chargement. Ainsi le système de traitement est indépendant du chargement. Le traitement peut intervenir à toutes les étapes du transport du conteneur, comme sur une plateforme portuaire. Plusieurs conteneurs peuvent être traités, par exemple sur le quai, avant leur embarquement. Des conteneurs importés peuvent aussi être traités, après avoir été débarqués, avant leur réexpédition.

Un système selon l'invention est mobile et ne reste pas fixé au conteneur. Ainsi, dans un exemple de mise en œuvre, un conteneur est chargé de ses marchandises chez leur expéditeur, puis livré sur une zone de traitement ou un système mobile de traitement est installé sur le conteneur, le temps du traitement, puis retiré pour effectuer un traitement dans un autre conteneur.

## Revendications

1. Procédé pour un traitement thermique à l'aide d'un gaz d'une marchandise (8) dans un conteneur (1) de type multimodal, ledit conteneur comprenant un sol (2), un toit (3), deux parois latérales et longitudinales (4), opposées entre elles, un fond (5), disposé transversalement entre les parois latérales, et, une entrée (6E) opposée audit fond (5), ladite entrée comprenant de préférence une porte droite (6D) et une porte gauche (6G), **caractérisé en ce qu'**il comprend la fourniture d'un système (9) et sa disposition de façon amovible dans un encadrement de ladite entrée, ce système comprenant au moins deux buses d'angle (14 ; 30C) et la disposition de chacune desdites buses d'angle à proximité d'un angle formé par une paroi latérale (4) respective avec le sol (2) et orientées de façon à générer des flux dudit gaz sensiblement longitudinaux, c'est-à-dire dirigés vers ledit fond (5) dudit conteneur.

2. Procédé selon la revendication 1, **caractérisé en ce qu'**il comprend la fourniture d'au moins une buse basse (11,16 ; 30D) et la disposition de ladite au moins une buse basse à proximité du sol (2), entre les buses d'angle.

3. Procédé selon l'une des revendications 1 et 2, **caractérisé en ce qu'**il comprend la fourniture d'au moins une buse haute (16, 12) et la disposition de ladite au moins une buse haute à proximité du toit (3).

4. Procédé selon l'une des revendications 1 à 3, **caractérisé en ce que** les buses sont disposées de sorte qu'elles émettent un flux (FA, FB, FH, FX ; F) sensiblement horizontalement et longitudinalement en direction du fond (5) du conteneur (1).

5. Procédé selon l'une des revendications 1 à 4, **caractérisé en ce que** les buses sont disposées à proximité ou au voisinage immédiat d'un encadrement de l'entrée (6E).

6. Procédé selon l'une des revendications 1 à 5, **caractérisé en ce qu'**il est appliqué au traitement thermique d'une marchandise qui comprend des grumes (8).

7. Procédé selon l'une des revendications 1 à 6, **caractérisé en ce que** les buses sont disposées sur un portique (10) qui assure une connexion fluidique entre elles et au moins un tuyau d'alimentation en gaz, de préférence de la vapeur d'eau, ledit portique étant disposé à l'intérieur du conteneur, à proximité de l'entrée (6E).

8. Système de traitement (9) pour la mise en œuvre d'un procédé selon la revendication 7, **caractérisé en ce qu'**il comprend un portique (10), ledit portique comprenant au moins une rampe basse (11) et une rampe haute (12) fluidiquement reliées entre elles par un montant (13), chaque rampe comprenant au moins une buse (16), une buse d'angle (16) étant fluidiquement reliée à ladite rampe basse, de préférence à la jonction de ladite rampe basse et dudit montant (13).

9. Système selon la revendication 8, **caractérisé en ce que** le portique comprend une rampe haute (12) et deux rampes basses (11), chaque rampe basse étant fluidiquement reliée à une extrémité respective de ladite rampe haute par un montant (13) respectif, une buse d'angle (16) respective étant fluidiquement reliée à chaque rampe basse, de préférence à la jonction de ladite rampe basse et de son montant (13) respectif.

10. Système selon l'une des revendications 8 ou 9, **caractérisé en ce que** chaque rampe (11, 12) comprend au moins trois buses (16).

11. Système selon l'une des revendications 8 à 10, **caractérisé en ce que** chaque buse d'angle est formée à une extrémité distale d'une tuyère (14), ladite tuyère étant prévue pour s'étendre longitudinalement, dans l'angle formé par le sol (2) et une paroi latérale (4) respective.

12. Système selon l'une des revendications 8 à 11, **caractérisé en ce qu'**il comprend en outre un panneau (17) pour la fermeture du conteneur (1) pendant le traitement, en remplacement d'une porte du conteneur (6G) maintenue ouverte.

13. Système selon la revendication 12, caractérisé en ce le panneau (17) comprend au moins un passage pour au moins un tuyau (19) pour alimenter le portique en gaz, et/ou, une bouche d'extraction (21).

14. Système de traitement (29) pour la mise en œuvre d'un procédé selon l'une des revendications 1 à 7, **caractérisé en ce qu'**il comprend au moins une buse prévue pour être insérée dans un interstice autour d'une porte du conteneur.

15. Système selon la revendication 14, **caractérisé en ce que** la buse comprend un diffuseur (34) prévu pour être inséré dans l'interstice et un emmanchement (32) prévu pour y fixer un tuyau d'alimentation en gaz, de préférence de la vapeur, ledit emmanchement étant prévu pour être à l'extérieur du conteneur, et, de préférence, une chambre d'admission qui relie fluidiquement l'emmanchement et le diffuseur.
